(19) Europäisches Patentamt
European Patent Office
Office européen des brevets

(11) Publication number : **0 493 926 A1**

(12) **EUROPEAN PATENT APPLICATION**

(21) Application number : **91311690.1**

(22) Date of filing : **17.12.91**

(51) Int. Cl.$^5$ : **C12N 15/68,** C12N 15/70, C12N 15/18

(30) Priority : **19.12.90 US 629803**

(43) Date of publication of application : **08.07.92 Bulletin 92/28**

(84) Designated Contracting States : **AT BE CH DE DK ES FR GB GR IT LI NL SE**

(71) Applicant : **ELI LILLY AND COMPANY**
**Lilly Corporate Center**
**Indianapolis Indiana 46285 (US)**

(72) Inventor : **Hershberger, Charles Lee**
**722 South 600 West**
**New Palestine, Indiana 46163 (US)**
Inventor : **Rosteck, Paul Robert, Jr.**
**4247 Saffron Drive**
**Indianapolis, Indiana 46237 (US)**

(74) Representative : **Hudson, Christopher Mark et al**
**Erl Wood Manor**
**Windlesham Surrey GU20 6PH (GB)**

(54) **A method for enhancing the structural stability of recombinant DNA expression vectors.**

(57) This invention provides a method of conferring structural stability upon an expression vector which enables the production of a heterologous polypeptide from a host cell. The method comprises lowering the copy number of the expression vector within he host cell to a copy number at which the vectors are structurally stable.

EP 0 493 926 A1

This invention relates to a method for enhancing the structural stability of recombinant DNA expression vectors. Loss of the structural stability of recombinant DNA expression vectors results in DNA deletions and rearrangements that alter vector structure. This is a significant concern in large scale cultures grown to produce heterologous polypeptides encoded by these expression vectors. The structure of these vectors is often altered such that expression of the encoded heterologous polypeptide is prevented. Thus, when the cultures are induced to express the heterologous polypeptide, a negative selective pressure toward a lack of expression of the heterologous polypeptide results in accumulation of the altered expression vectors (Ensley, B.D., 1986, CRC Crit. Rev. Biotechnol. 4 (3):263).

In view of the above, regulatory agencies, such as the Food and Drug Administration, require full characterization of recombinant DNA expression vectors that are utilized to produce heterologous proteins. Evidence must be submitted to verify that the recombinant DNA expression vector has the same structure at the end of fermentation as the expression vector from the original inoculum.

Four types of vector instability have been recognized (Ensley, B.D., supra). Segregational instability causes the loss of the vector from the host cell by failure to distribute at least one copy of the vector to each progeny of host cell division. Insertional instability causes insertion sequences and transposons to move into the vector DNA. Sequence instability causes base pair substitutions and frameshifts to accumulate in the vector DNA sequence. Structural instability causes deletions and DNA rearrangements which alter the vector structure or nucleotide sequence. The present invention concerns structural instability.

In the large scale fermentation of heterologous polypeptide products by recombinant DNA techniques, expression vectors are frequently constructed in a manner that allows for a maximum number of copies of the expression vectors within the host cell. The expectation is that because higher expression vector copy number provides a high dosage of the gene encoding the polypeptide of interest within the host cell, the yield of the desired heterologous polypeptide will be higher. However, these high copy number vectors are often plagued by structural instability (Nugent et al., 1985, Dev. Ind. Microbiol. 24:271). This instability results in the accumulation of structurally altered expression vectors, many of which are unable to produce the desired heterologous polypeptide product. These unstable vectors are detected upon analysis of the structure of vector DNA from a sample of the host cells at the end of fermentation.

The present invention provides a method of conferring structural stability upon expression vectors encoding heterologous polypeptides. The method comprises lowering the copy number of the expression vector within the host cell to a copy number at which the expression vector is structurally stable.

For the purposes of the present invention, as disclosed and claimed herein, the following terms are defined below:

ApR - the ampicillin resistant phenotype or gene conferring the same.

cI857 - the gene encoding a temperature sensitive form of the bacteriophage lambda cI repressor.

Copy control element - an element that, when inserted into a recombinant DNA vector, functions to lower the copy number of the recombinant DNA vector.

EK-Bovine Growth Hormone - methionyl-phenylalanyl-(aspartyl)4-lysinyl-bovine growth hormone, with indicated amino terminal amino acid sequence phenylalanyl-(aspartyl)4-lysinyl specifying an enterokinase cleavage site.

EK-BGH - EK-bovine growth hormone.

Functional polypeptide - a recoverable, biologically active heterologous or homologous polypeptide or precursor, a recoverable bioactive polypeptide comprised of a heterologous polypeptide and a partial or whole homologous polypeptide, or a recoverable bioinactive polypeptide containing a heterologous polypeptide and a bioinactivating polypeptide which can be specifically cleaved or a bioinactive polypeptide that can be converted to a bioactive polypeptide by refolding or other chemical treatments known to those skilled in the art.

pL - bacteriophage lambda leftward promoter.

Promoter - a DNA sequence that directs transcription of DNA into RNA in a regulatable fashion.

Recombinant DNA expression Vector - any recombinant DNA cloning vector into which a promoter has been incorporated.

TetR - the tetracycline resistant phenotype or gene conferring the same.

Transcriptional Activating Sequence - a DNA sequence that directs the transcription of DNA in a regulatable fashion.

Translational Activating Sequence - any DNA sequence, including the ribosome binding site and translational start codon, such as 5′-ATG-3′, that provides for the initiation of translation of a mRNA transcript into a peptide or polypeptide.

Figure 1 is a restriction and function map of pCZR125.

Figure 2 is a restriction and function map of pPR12.

Figure 3 is a restriction and function map of pHPR91.

This invention provides a method of conferring structural stability upon an expression vector which enables the production of a heterologous polypeptide from a host cell, said method comprising lowering the copy number of said expression vector within said host cell to a copy number at which said vector is structurally stable; wherein the gene encoding the heterologous polypeptide is expressed from a regulatable promoter.

A preferred method of the invention is that wherein the copy number of the expression vector is lowered by insertion of the DNA encoding the rop gene into the expression vector. In this embodiment, the rop gene is incorporated into an expression vector which preferably contains the pMB1 origin of replication; other origins of replication from the ColEl family of replicons are also useful. The product of the rop gene lowers the copy number of the expression vector to about 15-30 copies per host cell. The lower copy number of the expression vector within the host cell results in enhanced expression vector stability.

The vector ColEl is the best characterized of a group of vectors that use similar replication machinery. The vectors in the series include pMB1, ColEl, CloDF13, RSF1030, NTP1 and p15A (Balbas et al., 1986, Gene, 50:3; Selzer et al., 1983, Cell 32:119). These vectors are replicated by DNA polymerase I, encoded by the host cell polA gene. Replication begins from a 555 base RNA primer that is transcribed as a precursor of the vector primer by RNA polymerase and processed by RNAse H to expose the 3′ end where DNA synthesis initiates (Cesareni et al., 1984, EMBO J. 3:1365; Chan et al., 1985, J. Biol. Chem. 260:8925; Itoh et al. 1980, Proc. Natl. Acad. Sci. USA 77:2450). RNA I interacts with the precursor of the primer to inhibit formation of the precursor RNA-DNA hybrid needed to initiate DNA replication (Tomizawa et al., 1981, Proc. Natl. Acad. Sci. USA 78:6096; Tomizawa et al., 1981, Proc. Natl. Acad. Sci. USA 78:1421). Thus, the interaction of RNA I exerts negative control of vector replication thereby modulating the vector cony number. The protein product of the vector rop gene (also called the rom gene (Tomizawa et al., 1984, Cell 38: 871)) facilitates the interaction of RNA I and the primer. Therefore, the rop protein exerts additional negative control of the vector copy number.

The rop gene has been removed from ColEl type expression vectors to relieve the negative copy number control exerted by the rop gene product (Cesareni et al., 1982, Proc. Natl. Sci. USA 79:6313-6317). Copy number of these expression vectors is increased to about 100-300 copies per host cell. Therefore, the gene dosage of the gene encoded by the expression vector is increased. The expectation is that the higher gene dosage will increase the yield of the encoded protein. However, when these cultures are grown in large volumes, the rop expression vectors are structurally unstable. This instability results in the accumulation of structurally altered expression vectors. Many of the expression vectors are altered such that the desired polypeptide product is not produced. These structurally altered expression vectors are detected upon isolation of the expression vector DNA from the cells of the large scale cultures and analysis by gel electrophoresis techniques.

Additionally, vectors which do not contain a ColEl type origin of replication are useful in the method of the present invention. Structural stability can be conferred on high copy number vectors which are structurally unstable by insertion of a copy number control element into the vector. For example, structural stability can be provided to R1-type vectors (Womble and Rownd, 1988, Microbiol. Rev. 52(4):433 and Persson et al., 1988, EMBO J. 7(10):3279) which have been altered such that the copy number has been increased. In this case, structural stability is provided by insertion of a functional cop gene into the vector. The cop gene lowers the copy number of the vector, and provides structural stability.

Other methods for providing expression vector copy number control and, thus, vector structural stability, include mutating the host cell into which the expression vector is transformed such that a lower vector copy number results. Expression vector copy number control may also be provided by mutating the expression vector DNA such that a lower copy number results. Methods for deriving these copy number control mutations are described by Beckwith et al., 1986, Mol. Gen. Genet. 205:285.

Briefly, spontaneous mutants of the expression vector or the host cell which provide lower expression vector copy number are isolated as follows. Host cells containing the expression vector can be cultured overnight in an appropriate growth medium and then plated on non-selective media. Structurally unstable vectors are often found to have lost resistance to antibiotics due to loss of a portion of DNA of the expression vector which encodes the antibiotic resistance gene. The resultant colonies can be screened on plates containing antibiotic. Colonies are identified that retain the antibiotic resistance gene. These colonies are analyzed for vector copy number to see if they possess a lower vector copy number than those cells that had lost resistance to the antibiotic.

To determine whether the host cell or the expression vector was mutated, the expression vector is cured from one of the low copy number colonies. This clone is then retransformed with the parental vector. If the copy number of this parental vector is low, the copy number control can be attributed to a host cell mutation. If the copy number of the parental vector is high, then the copy number control mutation can be attributed to a mutation present on the cured vector.

Alternatively, purified vector can be transformed into new host cells. If the vector copy in these new host cells is low then the mutation in copy number control can be attributed to the vector; however, if the copy number is normal, then the mutation can be attributed to the original host cell.

The vector copy number must be lowered to a number which allows for the structural stability of the vector. Structural stability of the expression vector is analyzed as described in Example 3. The copy number of the expression vector within the host cell can be analyzed by methods well known in the art (Fitzwater et al.,1988, EMBO J. 7(10):3289; Yamaguchi and Tomizawa, 1980, Mol. Gen. Genet. 178:525).

The expression vectors useful in the method of the present invention contain DNA that encodes a heterologous polypeptide. The expression of the heterologous polypeptide is under the control of a functionally linked, regulatable promoter. The method of the present invention will be useful in conferring structural stability to expression vectors that enable expression of a wide variety of heterologous polypeptides. Examples of such polypeptides include: human insulin A-chain; human insulin B-chain; human proinsulin; human insulin A-chain, B-chain and proinsulin analogs; human growth hormone; bovine growth hormone; EK-bovine growth hormone; insulin-like growth factors; human transferrin; human interferons and human tissue plasminogen activators and derivatives thereof.

A preferred group of these heterologous polypeptides comprises: human insulin A-chain, B-chain and proinsulin analogs; human growth hormone; bovine growth hormone; EK-bovine growth hormone; insulin-like growth factors; human transferrin; human interferons; and human tissue plasminogen activators and derivatives thereof.

Another preferred group of heterologous polypeptides comprises: human growth hormone; bovine growth hormone; EK-bovine growth hormone; insulin-like growth factors; human transferrin; human interferons; and human tissue plasminogen activators and derivatives thereof.

Another preferred group of heterologous polypeptides comprises: human growth hormone; EK-bovine growth hormone and insulin-like growth factors. Another preferred heterologous polypeptide is EK-bovine growth hormone.

The preferred expression vectors useful in the method of the present invention additionally contain a selective marker such as an antibiotic resistant gene. This allows the selective growth of those cells that contain the vector DNA in the presence of antibiotic. Those skilled in the art will recognize that a variety of selective markers may be used to provide selective pressure for those host cells that contain the desired expression vector.

Vectors useful in the method of the present invention comprise a regulatable promoter, such as the Escherichia coli bacteriophage lambda pL promoter-operator region, which enables the transcription of a functionally linked, heterologous gene. When using an expression vector which contains the lambda pL promoter-operator region in the method of the present invention, the most preferred among these promoters are the bacteriophage lambda pL promoter-operator regions disclosed by U.S. Patent Application Serial No. 07/565,783, filed August 13, 1990. These lambda promoter-operator regions comprise modifications such that the DNA sequence located in a position 5′ to the repressor binding region has been deleted. These modified lambda promoter-operator regions provide regulatable expression of a functionally linked, heterologous gene and provide enhanced structural stability of the recombinant DNA vector as compared to expression vectors which contain the wild type bacteriophage lambda promoter-operator region.

The preferred lambda pL operator-promoter regions comprise the following structures:

The preferred transcriptional activating sequences of the present invention have the following structures:

```
5'-CATACAGATAACCATCTGCGGTGATAAATTATCTCTGGCGGTGTTGACA
   |||||||||||||||||||||||||||||||||||||||||||||||||
3'-GTATGTCTATTGGTAGACGCCACTATTTAATAGAGACCGCCACAACTGT

TAAATACCACTGGCGGTGATACTGAGCACATCA-3'
|||||||||||||||||||||||||||||||||
ATTTATGGTGACCGCCACTATGACTCGTGTAGT-5';


5'-CATACAGATAACCATCTGCGGTGATAAATTATCTCTGGCGGTGTTGACA
   |||||||||||||||||||||||||||||||||||||||||||||||||
3'-GTATGTCTATTGGTAGACGCCACTATTTAATAGAGACCGCCACAACTGT

TAAATACCACTGGCGGTGGTACTGAGCACATCA-3'
|||||||||||||||||||||||||||||||||
ATTTATGGTGACCGCCACCATGACTCGTGTAGT-5';
```

```
5'-CATACAGATAACCATCTGCGGTGATAAATTATCTCTGGCGGTGTTGACA
   |||||||||||||||||||||||||||||||||||||||||||||||||
3'-GTATGTCTATTGGTAGACGCCACTATTTAATAGAGACCGCCACAACTGT

TAAATACCACTGGCGGTTATAATGAGCACATCA-3'
|||||||||||||||||||||||||||||||||
ATTTATGGTGACCGCCAATATTACTCGTGTAGT-5';

5'-CAAAAAATAAATTCATATAAAAAACATACAGTTAACCATCTGCGGTG
   |||||||||||||||||||||||||||||||||||||||||||||||
3'-GTTTTTTATTTAAGTATATTTTTTGTATGTCAATTGGTAGACGCCAC

ATAAATATTTATCTCTGGCGGTGTTGACATATACCACTGGCGGTGATATAATGA
||||||||||||||||||||||||||||||||||||||||||||||||||||||
TATTTATAAATAGAGACCGCCACAACTGTATATGGTGACCGCCACTATATTACT

GCACATCA-3'
||||||||
CGTGTAGT-5';

5'-CAAAAAATAAATTCATATAAAAAACATACAGTTATTTATCTCTGG
   |||||||||||||||||||||||||||||||||||||||||||||
3'-GTTTTTTATTTAAGTATATTTTTTGTATGTCAATAAATAGAGACC

   CGGTGTTGACATAAATACCACTGGCGGTTATAATGAGCACATCA-3'
   ||||||||||||||||||||||||||||||||||||||||||||
   GCCACAACTGTATTTATGGTGACCGCCAATATTACTCGTGTAGT-5';
```

and

wherein

A is deoxyadenyl;
G is deoxyguanyl;
C is deoxycytidyl;
T is thymidyl.

Single stranded deoxyoligonucleotides encoding both strands of the transcriptional activating sequences can be synthesized with commercially available instruments such as the 380B DNA synthesizer marketed by Applied Biosystems (850 Lincoln Center Dr., Foster City CA 94404), using β-cyanoethyl phosphoramidite chemistry. Other procedures for synthesizing DNA are also known in the art. The conventional modified phosphotriester method of synthesizing single stranded DNA is described by Itakura, et al., 1977, Science 198:1056 and by Crea, et al., 1978 Proc. Nat. Acad. Sci. U.S.A. 75:5765, and can also be used to synthesize the DNA encoding the transcriptional activating sequences.

A number of host cells can be used in the method of the present invention. Such host cells include Escherichia coli K12 RV308, E. coli MM294, E. coli K12 C600, E. coli K12 DH5 alpha, and E. coli K12 JM109. The skilled artisan will recognize that other host cells are available for the practice of the present invention and that the scope of the present method is not limited to the host cells presented herein.

The plasmid pCZR125 is illustrative of the starting recombinant DNA expression vectors of the present invention. This plasmid was constructed using the plasmid pL110 as starting material. Plasmid pL110 is disclosed and claimed by U.S. Patent 4,874,703, issued October 17, 1989, which is incorporated herein by reference. The plasmid pCZR125 was constructed by ligating the 5.8 kb XbaI-BamHI fragment of pL110 with two synthetic DNA fragments as described in Example 1. The synthetic DNA fragments encode an intact first cistron as well as the EK-bGH gene. The two-cistron expression system is described by Schoner et al. 1986, Proc. Natl. Acad. Sci. USA 83:8506.

The plasmid pHPR91 is illustrative of the plasmids useful in the method of the present invention. Plasmid pHPR91 was constructed by ligation of the 5051 base pair AvaI restriction fragment of plasmid pPR12 withthe 1876 base pair EcoRI-ScaI restriction fragment of plasmid pCZR125. The AvaI restriction fragment of pPR12 encodes the rop gene. Plasmid pHPR91 is a structurally stable vector which provides expression of the EK-bGH gene.

The modified pL promoter-operator regions, described above, can be synthesized with appropriate cohesive ends which allow their cloning into a position within the expression vector that allows expression of the heterologous gene. For example, these promoters can be synthesized with EcoRI-BglII cohesive ends. These EcoRI-BglII tailed promoters can be cloned into the 6.51 kilobase pair EcoRI-BglII restriction fragment of pHR91. In this manner the modified pL promoter replaces the wild type lambda pL promoter present in pHPR91.

Those skilled in the art will further recognize that the present invention provides a method wherein a heterologous polypeptide product is expressed by a host cell using appropriate fermentation conditions. Upon completion of fermentation, the heterologous polypeptide product is isolated from the fermentation broth by a variety of methods known in the art. The present invention can be practiced in both defined and complex media formulations. Those skilled in the art recognize that the medium used in the fermentation process is critical to maximize product expression. A variety of media including, for example, those discussed by Maniatis et al., Molecular Cloning: A Laboratory Manual, Cold Spring Harbor Laboratory Press, Cold Spring Harbor, N.Y. (2d ed. 1989), should be evaluated to determine the best medium for the particular expression system used in practicing the invention. For example, a medium formulation such as that described by Domach et al., 1984, Biotechnology and Bioengineering vol. XXVI:203-206, can be used as the fermentation medium for growth of the host cells. The host cells are cultured under condition suitable for growth until numbers sufficient for optimal expression are obtained. At this time the regulatable promoter that drives expression of the heterologous gene can be derepressed and, thus, allow the expression of the heterologous gene product to occur.

The following examples are intended to assist in further understanding of the invention. Particular materials employed, species, and conditions are intended to be further illustrative of the scope thereof. All enzymes referred to in the examples are available, unless otherwise indicated, from Bethesda Research Laboratories (BRL), Gaithersburg, MD 20877 or New England Biolabs Inc. (NEB), Beverly, MA 01915, or Boehringer-Manneheim Biochemicals, 7941 Castleway Drive, P.O. Box 50816, Indianapolis Indiana 46250 and are used in substantial accordance with the manufacturer's recommendations.

## Example 1

## Construction of pCZR125

### A. Preparation of the 5.8 kb XbaI-BamHI Restriction Fragment of pL110

Twenty-five μg of plasmid pL110 was digested to completion with 15 μl (150 units) of XbaI in a 500 μl reaction volume containing 60 mM Tris-HCl (pH 7.5) (Tris is Tris[hydroxymethyl]aminomethane), 10 mM $MgCl_2$, 100 mM $NaCl_2$ and 1 mM β-mercaptoethanol. The mixture was incubated at 37°C for one hour. The digested DNA was extracted two times with a mixture of phenol and chloroform (50:50) and the aqueous layer was recovered. The DNA was recovered from the aqueous layer by addition of 2.5 volumes of absolute ethanol and 0.1 volume of 3 M sodium acetate. The DNA was collected by centrifugation and was resuspended in 50 μl of water.

The above DNA was partially digested with BamHI as follows. Fifty μl of the XbaI-digested DNA was mixed with 0.2 μl (2 units) of BamHI in a 150 μl reaction volume consisting of 10 mM Tris-HCl (pH 7.8), 7 mM $MgCl_2$, 150 mM NaCl, and 6 mM β-mercaptoethanol. The mixture was incubated at 37°C for 5 minutes. The sample was purified and recovered as described above and resuspended in 50 μl of TE (TE is 10 mM Tris-HCl (pH 7.4) and 1 mM ethylenediaminetetra-acetic acid (EDTA)). Five μl of loading buffer (25% v/v glycerol, 0.05% w/v bromphenol blue, and 0.5% w/v xylene cyanole) was added to the sample and the digested DNA was fractionated on a 1% agarose gel by gel electrophoresis as described by Maniatis et al. at pages 150-172 (Maniatis et al., 1982, Molecular Cloning: a Laboratory Manual, Cold Spring Harbor Laboratory, Cold Spring Harbor, N.Y.). The agarose gel was stained with a dilute solution of ethidium bromide and the 5.8 kb XbaI-BamHI restriction fragment was visualized under a 300nm UV light. The portion of the gel containing this restriction fragment was recovered. The DNA was purified by mincing the gel slice, extracting twice with phenol:chloroform (50:50) and ethanol precipitating the DNA as described above.

### B. Preparation of XbaI-NdeI linker

The following complementary DNA segments were synthesized on an automated DNA synthesizer (Applied Biosystems 380B) using β-cyanoethyl phosphoramidite chemistry:

```
     1.   5'-CTAGAGGGTATTAATAATGTATATTGATTTTAATAAGGA
GGAATAATCA-3' (SEQ ID NO:  1)
     2.   5'-TATGATTATTCCTCCTTATTAAAATCAATATACATTATT
AATACCCT-3' (SEQ ID NO:  2).
```

These single stranded DNA segments were conventionally purified and resuspended in water.

Five μg of each single stranded DNA segment was mixed and heated to 70°C for five minutes. The mixture was cooled at room temperature for 30 minutes to allow the DNA segments to anneal.

The annealed DNA fragment was treated with 1 μl (10 units) of T4 polynucleotide kinase in 70 mM Tris-HCl (pH 7.6), 0.1 M KCl, 10 mM $MgCl_2$, 5 mM DTT containing 0.2 mM adenine 5′-triphosphate in a total volume of 20 μl. The mixture was incubated at 37°C for thirty minutes. The mixture was then incubated at 70°C for 5 minutes and then cooled at room temperature.

C. Preparation of the Synthetic EK-BGH gene

The DNA fragment encoding the EK-BGH gene was synthesized in substantial accordance with the method of Example 1B. The gene encoding EK-BGH was constructed from 16 chemically synthesized pieces of single stranded DNA, ranging from 71 to 83 nucleotides in length, which, when annealled, comprise both complementary strands of the EK-BGH gene with NdeI-BamHI cohesive ends. The sequence (SEQ ID NO: 3) of the synthetic EK-BGH gene is:

```
5'-TATGTTCCCATTGGATGATGATGATAAGTTCCCAGCCATGTCCTT
     |||||||||||||||||||||||||||||||||||||||||||
  3'-ACAAGGGTAACCTACTACTACTATTCAAGGGTCGGTACAGGAA

GTCCGGCCTGTTTGCCAACGCTGTGCTCCGGGCTCAGCACCTGCATCAGCTGGCTGCTGA
||||||||||||||||||||||||||||||||||||||||||||||||||||||||||||
CAGGCCGGACAAACGGTTGCGACACGAGGCCCGAGTCGTGGACGTAGTCGACCGACGACT

CACCTTCAAAGAGTTTGAGCGCACCTACATCCCGGAGGGACAGAGATACTCCATCCAGAA
||||||||||||||||||||||||||||||||||||||||||||||||||||||||||||
GTGGAAGTTTCTCAAACTCGCGTGGATGTAGGGCCTCCCTGTCTCTATGAGGTAGGTCTT

CACCCAGGTTGCCTTCTGCTTCTCTGAAACCATCCCGGCCCCCACGGGCAAGAATGAGGC
||||||||||||||||||||||||||||||||||||||||||||||||||||||||||||
GTGGGTCCAACGGAAGACGAAGAGACTTTGGTAGGGCCGGGGGTGCCCGTTCTTACTCCG
```

```
CCAGCAGAAATCAGACTTGGAGCTGCTTCGCATCTCACTGCTCCTCATCCAGTCGTGGCT
||||||||||||||||||||||||||||||||||||||||||||||||||||||||||||
GGTCGTCTTTAGTCTGAACCTCGACGAAGCGTAGAGTGACGAGGAGTAGGTCAGCACCGA

TGGGCCCCTGCAGTTCCTCAGCAGAGTCTTCACCAACAGCTTGGTGTTTGGCACCTCGGA
||||||||||||||||||||||||||||||||||||||||||||||||||||||||||||
ACCCGGGGACGTCAAGGAGTCGTCTCAGAAGTGGTTGTCGAACCACAAACCGTGGAGCCT

CCGTGTCTATGAGAAGCTGAAGGACCTGGAGGAAGGCATCCTGGCCCTGATGCGGGAGCT
||||||||||||||||||||||||||||||||||||||||||||||||||||||||||||
GGCACAGATACTCTTCGACTTCCTGGACCTCCTTCCGTAGGACCGGGACTACGCCCTCGA

GGAAGATGGCACCCCCCGGGCTGGGCAGATCCTCAAGCAGACCTATGACAAATTTGACAC
||||||||||||||||||||||||||||||||||||||||||||||||||||||||||||
CCTTCTACCGTGGGGGGCCCGACCCGTCTAGGAGTTCGTCTGGATACTGTTTAAACTGTG

AAACATGCGCAGTGACGACGCGCTGCTCAAGAACTACGGTCTGCTCTCCTGCTTCCGGAA
||||||||||||||||||||||||||||||||||||||||||||||||||||||||||||
TTTGTACGCGTCACTGCTGCGCGACGAGTTCTTGATGCCAGACGAGAGGACGAAGGCCTT

GGACCTGCATAAGACGGAGACGTACCTGAGGGTCATGAAGTGCCGCCGCTTCGGGGAGGC
||||||||||||||||||||||||||||||||||||||||||||||||||||||||||||
CCTGGACGTATTCTGCCTCTGCATGGACTCCCAGTACTTCACGGCGGCGAAGCCCCTCCG

CAGCTGTGCCTTCTAG-3'
||||||||||||||||
GTCGACACGGAAGATCCTAG-5'.
```

D. DNA Ligation

Two µl (0.2 µg) of the pL110 restriction fragment prepared in Example 1A, 2 µl (8.75 pmoles) of the DNA fragment prepared in Example 1B, and 2 µl (0.1 µg) of the DNA fragment prepared in Example 1C were ligated in a reaction containing 1 µl (10 units) of T4 DNA ligase, 50 mM Tris-HCl (pH 7.6), 10 mM $MgCl_2$, 1 mM dithiothreitol, 1 mM of adenosine 5′-triphosphate and 5% (w/v) polyethylene glycol-8000 in a total volume of 10 µl. The mixture was incubated at 16° for 16 hours. A portion of this mixture was used to transform Escherichia coli cells as described below.

E. Transformation Procedure

Escherichia coli K12 RV308 cells are available from the Northern Regional Research Laboratory, Peoria, Illinois under the accession number NRRL B-15624. A 50 ml culture of E. coli K12 RV308 was grown in L-broth (10 g tryptone, 10 g NaCl and 5 g yeast extract per liter of $H_20$) to an $O.D._{590}$ of 0.5 absorbance units. The culture was chilled on ice for ten minutes and then the cells were collected by centrifugation. The cell pellet was resuspended in 25 ml of cold 50 mM $CaCl_2$:10 mM Tris-HCl (pH 8.0) and incubated on ice for 15 minutes. The cells were collected by centrifugation, the cell pellet was resuspended in 2.5 ml of cold 50 mM $CaCl_2$:10mM Tris-HCl (pH 8.0) and the sample was held at 4°C for 16 hours.

Two hundred µl of this cell suspension was mixed with 50 µl of the ligated DNA prepared above and then incubated on ice for 60 minutes. The mixture was incubated at 32°C for 45 seconds and then placed on ice for 2 minutes. Five ml of TY medium (1% tryptone, 0.5% yeast extract and 1% sodium chloride, pH 7.4) was added to the mixture and incubation was continued at 32°C for 2 hours. One hundred µl of this culture was spread on TY agar plates (1% tryptone, 0.5% yeast extract, 1% sodium chloride and 1.5% agar at pH 7.4) that contained 5 µg/ml of tetracycline. These plates were incubated 16 hours at 32°C. The tetracycline resistant colonies were individually picked and used to inoculate 2 ml of TY medium. The cultures were incubated at 37°C with aeration for 16 hours.

F. DNA Isolation Procedure

Plasmid DNA was isolated from the culture of transformants as follows. All of the following manipulations were done at ambient temperature unless otherwise indicated. One and a half ml of each of the cultures was transferred to a microcentrifuge tube. The cells were collected by a 1 minute centrifugation. The supernatant was removed with a fine-tip aspirator and the cell pellet was suspended in 100 μl of a solution containing 50 mM glucose, 10 mM EDTA and 25 mM Tris-HCl (pH 8.0). After incubation at room temperature for 5 minutes, 200 μl of an alkaline sodium dodecyl sulfate (SDS) solution (0.2 N NaOH, 1% SDS) was added. The tube was gently inverted to mix and then maintained on ice for 5 minutes. Next, 150 μl of a potassium acetate solution (prepared by adding 11.5 ml of glacial acetic acid and 28.5 ml of water to 60 ml of 5 M potassium acetate. The resulting solution is 3 M with respect to potassium and 5 M with respect to acetate) was added and the contents of the tube mixed by gently vortexing. The sample was kept on ice for 5 minutes and then centrifuged for 10 minutes. The supernatant was transferred to a second centrifuge tube to which an equal volume of phenol (saturated with 0.1 M Tris (pH 8.0)) was added. The sample was mixed and then centrifuged for 5 minutes. The supernatant was collected and the phenol extraction was repeated. One ml of ice-cold absolute ethanol was added to the supernatant. The sample was mixed and held on dry ice until highly viscous, but not frozen solid. The DNA was then collected by a 5 minute centrifugation. The supernatant was removed by aspiration and 500 μl of 70% ethanol was added to the DNA pellet. The sample was gently vortexed to wash the pellet and centrifuged for 2 minutes. The supernatant was removed and the DNA pellet was dried under vacuum. The DNA was dissolved in 50 μl of TE (10 mM Tris-HCl (pH 8.0) and 1 mM EDTA) and stored at 4°C.

G. Large Scale DNA Isolation

Large amounts of pCZR125 plasmid DNA were isolated as follows. One liter of L broth containing 5 μg/ml tetracycline was inoculated with a colony of Escherichia coli RV308/pCZR125. The culture was grown at 32°C for 16 hours. The culture was centrifuged in a GSA rotor (Sorvall) at 6000 rpm for 5 minutes at 4°C. The resulting supernatant was discarded, and the cell pellet was washed in 40 ml of TES buffer (10 mM Tris-HCl (pH 7.5), 10 mM NaCl, and 1 mM EDTA) and then collected by centrifugation. The supernatant was discarded, and the cell pellet was frozen in a dry ice-ethanol bath and then thawed. The thawed cell pellet was resuspended in 10 ml of a solution of 25% sucrose and 50 mM EDTA. One ml of a 5 mg/ml lysozyme solution, 3 ml of 0.25 M EDTA (pH 8.0), and 100 μl of 10 mg/ml boiled RNAse A (available from Sigma Chemical Co., P.O. Box 14508, St. Louis, Mo.) were added to the solution, which was then incubated on ice for 15 minutes. Three ml of lysing solution (prepared by mixing 3 ml of 10% Triton X-100, 75 ml of 0.25M EDTA (pH 8.0), 15 ml of 1 M Tris-HCl (pH 8.0), and 7 ml of $H_2O$) were added to the lysozyme treated cells, mixed, and the resulting solution incubated on ice for another 15 minutes. The lysed cells were frozen in a dry ice-ethanol bath and then thawed.

The cellular debris was removed from the solution by centrifugation at 25,000 rpm for 40 minutes in a SW28.1 rotor (Beckman, Scientific Instrument Division, Campus Drive at Jamboree Blvd., Irvine, CA 92713) and by extraction with buffered phenol. About 30.44 g of CsCl and ~ 1 ml of a 5 mg/ml ethidium bromide solution were added to the cell extract, and then the volume of the solution was adjusted to 40 ml with TES buffer (10 mM Tris-HCl (pH 7.5), 10 mM NaCl and 1 mM EDTA). The solution was decanted into a VTi50 ultracentrifuge tube (Beckman), which was then sealed and centrifuged in a VTi50 rotor at 42,000 rpm for about 16 hours. The resulting plasmid band, visualized with ultraviolet light, was isolated and then placed in a Ti75 tube and rotor (Beckman) and centrifuged at 50,000 rpm for 16 hours. Any necessary volume adjustments were made using TES containing 0.761 g/ml CsCl. The plasmid band was again isolated, extracted with salt-saturated isopropanol to remove ethidium bromide, and diluted 1:3 with TES buffer. One volume of 3 M sodium acetate and two volumes of absolute ethanol were then added to the solution, which was then incubated for 16 hours at -20 C. The plasmid DNA was pelleted by centrifuging the solution in a SS34 rotor (Sorvall) for 15 minutes at 10,000 rpm. The plasmid DNA obtained by this procedure was suspended in TE buffer and stored at -20°C.

Example 2

Construction of pHPR91

A. Preparation of the 1876 Base Pair EcoRI-ScaI Restriction Fragment of pCZR125

Ten μg of plasmid pCZR125 were digested to completion with 30 units of EcoRI in a 100 μl reaction volume containing 100 μg/ml BSA (bovine serum albumin) 50 mM Tris-HCl (pH 8.0) 10 mM $MgCl_2$, 100 mM NaCl at 37°C for one hour. The sample was then incubated at 70°C for 10 minutes to inactivate the EcoRI.

EcoRI digested plasmid pCZR125 was made blunt-ended by treatment with DNA polymerase I (Klenow Fragment) as follows. Twenty-five μl of the above reaction was adjusted to a 50 μl reaction volume containing 250 μM dATP (deoxyadenosine 5′-triphosphate), 250 μM dCTP (deoxycytosine 5′-triphosphate) , 250 μM dGTP (deoxyguanadine 5′-triphosphate) , 250 μM TTP (thymidine 5′-triphosphate), 50 mM Tris-HCl (pH 7.8), 10 mM $MgCl_2$ 10 mM B-mercaptoethanol and 5 units of DNA polymerase I (Klenow Fragment). The sample was incubated at 37°C for 30 minutes to complete complementary synthesis of the single strand of restriction fragment overhang. Then the reaction mixture was incubated at 70°C for 15 minutes to inactivate the Klenow Fragment.

The EcoRI digested, Klenow treated pCZR125 plasmid DNA was then digested to completion with ScaI by incubation at 37°C for one hour in a 150 μl reaction volume containing 50 mM Tris-HCl (pH 8.0), 10 mM $MgCl_2$, 100 mM NaCl, 100 μg/ml BSA and 18 units ScaI. The ScaI was then thermally inactivated by incubating the sample at 70°C for 10 minutes.

B. Preparation of the 5051 Base Pair AvaI Restriction Fragment of pPR12

The construction of plasmid pPR12 is taught in U.S. Patent Number 4,436,815, which issued on March 13, 1984 and the teachings of which are herein incorporated by reference.

Ten μg of pPR12 was digested to completion with 30 units of AvaI for one hour at 37°C in a 100 μl reaction volume containing 100 μg/ml BSA, 50 mM Tris-HCl (pH 8.0), 10 mM $MgCl_2$ and 50 mM NaCl. AvaI was then thermally inactivated by incubation at 70°C for 15 minutes.

The AvaI digested plasmid pPR12 sample was made blunt ended as follows. Twenty-five μl of the above reaction was adjusted to a 50 μl reaction volume containing 250 μM dATP, 250 μM dCTP, 250 μM dGTP, 250 μM TTP, 50 mM Tris-HCl (pH 7.8), 10 mM $MgCl_2$, 10 mM B-mercaptoethanol and 5 units of DNA polymerase I (Klenow Fragment). The sample was incubated at 37°C for 30 minutes to complete complementary synthesis of the single strand of restriction fragment overhang, and then at 70°C for 15 minutes to inactivate the Klenow Fragment.

C. Final Construction of pHPR91

The DNA samples prepared in Examples 2A and 2B were copurified and ethanol precipitated as described in Example 1. The DNA was recovered by centrifugation, dried and resuspended in 10 μl of water. The DNA fragments were then ligated by incubating at 4°C overnight in a 40 μl reaction volume containing 50 mM Tris-HCl (pH 7.8), 10 mM $MgCl_2$, 5 mM DTT (dithiotreitol), 5% glycerol, 0.2 mM adenosine 5′-triphosphate and 40 units of DNA ligase.

A portion of the ligation mixture was used to transform Escherichia coli K12 MM294 cells in accordance with the procedure of Example 1E. E. coli K12 MM294 cells are available from the American Type Culture Collection, Rockville, Maryland 20852 under accession number ATCC 31446. The transformants were selected on L agar containing 10 μg/ml tetracycline. Individual colonies were picked and grown in L broth containing 10 μg/ml tetracycline. Tetracycline resistant transformants containing the desired plasmid pHPR91 were identified following plasmid purification by restriction enzyme analysis. Digestion of plasmid pHPR91 with PvuII yields a 1450 base pair fragment. A restriction site and function map of plasmid pHPR91 is presented in Figure 3.

Example 3

Analysis of Vector Stability

Analysis of expression vectors was carried out from restriction analysis of the vector isolated from the fermentation mixture at the end of fermentation. The use of restriction analysis to characterize expression vectors is well known in the art. The methodology for the generation and the interpretation of restriction endonuclease mapping is well known in the art. Maniatis et al., supra., provide a review of this subject.

The structural stability of the vector useful in the method of the present invention was analyzed by isolating the vector DNA from the host cells of the fermentation mixture at the end of the fermentation. This DNA was isolated by the DNA miniprep procedure in substantial accordance with the method presented in Example 1F.

Restriction enzyme analysis was carried out by digesting isolated vector DNA. As an example, plasmid pHPR91 was digested with the restriction enzyme BamHI. Approximately 0.25 μg of the isolated vector DNA was digested to completion with 1 μl (10 units) of BamHI in a 10 μl reaction volume containing 50 mM Tris-HCl (pH 8.0), 50 mM NaCl, and 10 mM $MgCl_2$. The mixture was incubated at 37°C for one hour. The digested DNA was fractionated on an agarose gel and visualized as described in Example 1A.

Based on the restriction map of the plasmid pHPR91, a BamHI restriction digest resulted in two DNA fragments of 4792 and 2140 base pairs, indicating that the plasmid remained structurally stable through the fermentation process.

SEQUENCE LISTING

(1) GENERAL INFORMATION:

(i) APPLICANT:Eli Lilly and Company.

(ii) TITLE OF INVENTION:Method for Enhancing the Structural Stability of Recombinant DNA Expression Vectors

(iii) NUMBER OF SEQUENCES:9

(iv) CORRESPONDENCE ADDRESS:

(A) ADDRESSEE:Mr. C. Mark Hudson

(B) STREET:Erl Wood Manor

(C) CITY:Windlesham

(D) STATE:Surrey GU20 6PH

(E) COUNTRY:United Kingdom

(v) COMPUTER READABLE FORM:

(A) MEDIUM TYPE:Diskette, 3.50 inch, 1.0 Mb storage

(B) COMPUTER:Macintosh

(C) OPERATING SYSTEM:Macintosh

(D) SOFTWARE:Microsoft Word

(vi) CURRENT APPLICATION DATA:

(A) APPLICATION NUMBER:91311690

(B) FILING DATE:17 December 1991

(vii) ATTORNEY/AGENT INFORMATION:

(A) NAME:Mr. C. Mark Hudson

(B) REGISTRATION NUMBER:307

(viii) TELECOMMUNICATION INFORMATION:

(A) TELEPHONE:0276 78441

(B) TELEFAX:0276 78306

(C) TELEX:858177

(2) INFORMATION FOR SEQ ID NO:1:

(i) SEQUENCE CHARACTERISTICS:

(A) LENGTH:82 nucleotides

(B) TYPE:nucleic acid

(C) STRANDEDNESS:double

(D) TOPOLOGY:linear

(ii) MOLECULE TYPE:DNA

(xi) SEQUENCE DESCRIPTION: SEQ ID NO:1

CATACAGATA ACCATCTGCG GTGATAAATT ATCTCTGGCG GTGTTGACAT 50
AAATACCACT GGCGGTGATA CTGAGCACAT CA 82

(2) INFORMATION FOR SEQ ID NO:2:

(i) SEQUENCE CHARACTERISTICS:

(A) LENGTH:82 nucleotides

(B) TYPE:nucleic acid

(C) STRANDEDNESS:double

(D) TOPOLOGY:linear

(ii) MOLECULE TYPE:DNA

(xi) SEQUENCE DESCRIPTION: SEQ ID NO:2

CATACAGATA ACCATCTGCG GTGATAAATT ATCTCTGGCG GTGTTGACAT 50
AAATACCACT GGCGGTGGTA CTGAGCACAT CA 82

(2) INFORMATION FOR SEQ ID NO:3:

(i) SEQUENCE CHARACTERISTICS:

(A) LENGTH:82 nucleotides

(B) TYPE:nucleic acid
(C) STRANDEDNESS:double
(D) TOPOLOGY:linear
(ii) MOLECULE TYPE:DNA
(xi) SEQUENCE DESCRIPTION: SEQ ID NO:3
CATACAGATA ACCATCTGCG GTGATAAATT ATCTCTGGCG GTGTTGACAT 50
AAATACCACT GGCGGTTATA ATGAGCACAT CA 82

(2) INFORMATION FOR SEQ ID NO:4:
(i) SEQUENCE CHARACTERISTICS:
(A) LENGTH:110 nucleotides
(B) TYPE:nucleic acid
(C) STRANDEDNESS:double
(D) TOPOLOGY:linear
(ii) MOLECULE TYPE:DNA
(xi) SEQUENCE DESCRIPTION: SEQ ID NO:4
CAAAAAATAA ATTCATATAA AAAACATACA GTTAACCCAT CTGCGGTGAT 50
AAATATTTAT CTCTGGCGGT GTTGACATAT ACCACTGGCG GTGATATAAT 100
GAGCACATCA 110

(2) INFORMATION FOR SEQ ID NO:5:
(i) SEQUENCE CHARACTERISTICS:
(A) LENGTH:89 nucleotides
(B) TYPE:nucleic acid
(C) STRANDEDNESS:double
(D) TOPOLOGY:linear
(ii) MOLECULE TYPE:DNA
(xi) SEQUENCE DESCRIPTION: SEQ ID NO:5
CAAAAAATAA ATTCATATAA AAAACATACA GTTATTTATC TCTGGCGGTG 50
TTGACATAAA TACCACTGGC GGTTATAATG AGCACATCA 89

(2) INFORMATION FOR SEQ ID NO:6:
(i) SEQUENCE CHARACTERISTICS:
(A) LENGTH:48 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION: SEQ ID NO:6
TAGAGGGTA TTAATAATGT ATATTGATTTT AATAAGGAGG AATAATCA 48

(2) INFORMATION FOR SEQ ID NO:7:
(i) SEQUENCE CHARACTERISTICS:
(A) LENGTH:47 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION: SEQ ID NO:7
TATGATTATT CCTCCTTATT AAAATCAATA TACATTATTA ATACCCT 47

(2) INFORMATION FOR SEQ ID NO:8:
(i) SEQUENCE CHARACTERISTICS:
(A) LENGTH:601 nucleotides

(B) TYPE:nucleic acid
(C) STRANDEDNESS:double
(D) TOPOLOGY:linear
(ii) MOLECULE TYPE:DNA
(xi) SEQUENCE DESCRIPTION: SEQ ID NO:8

```
T ATG TTC CCA TTG GAT GAT GAT GAT AAG TTC CCA GCC ATG 40
TCC TTG TCC GGC CTG TTT GCC AAC GCT GTG CTC CGG GCT 79
CAG CAC CTG CAT CAG CTG GCT GCT GAC ACC TTC AAA GAG 118
TTT GAG CGC ACC TAC ATC CCG GAG GGA CAG AGA TAC TCC 157
ATC CAG AAC ACC CAG GTT GCC TTC TGC TTC TCT GAA ACC 196
ATC CCG GCC CCC ACG GGC AAG AAT GAG GCC CAG CAG AAA 235
TCA GAC TTG GAG CTG CTT CGC ATC TCA CTG CTC CTC ATC 274
CAG TCG TGG CTT GGG CCC CTG CAG TTC CTC AGC AGA GTC 313
TTC ACC AAC AGC TTG GTG TTT GGC ACC TCG GAC CGT GTC 352
TAT GAG AAG CTG AAG GAC CTG GAG GAA GGC ATC CTG GCC 391
CTG ATG CGG GAG CTG GAA GAT GGC ACC CCC CGG GCT GGG 430
CAG ATC CTC AAG CAG ACC TAT GAC AAA TTT GAC ACA AAC 469
ATG CGC AGT GAC GAC GCG CTG CTC AAG AAC TAC GGT CTG 508
CTC TCC TGC TTC CGG AAG GAC CTG CAT AAG ACG GAG ACG 547
TAC CTG AGG GTC ATG AAG TGC CGC CGC TTC GGG GAG GCC 586
AGC TGT GCC TTC TAG 601
```

(2) INFORMATION FOR SEQ ID NO:9:
(i) SEQUENCE CHARACTERISTICS:
(A) LENGTH:603 nucleotides
(B) TYPE:nucleic acid
(C) STRANDEDNESS:double
(D) TOPOLOGY:linear
(ii) MOLECULE TYPE:DNA
(ix) FEATURES:Reverse complement of SEQ ID NO:8
(xi) SEQUENCE DESCRIPTION: SEQ ID NO:9

```
GATC CTA GAA GGC ACA GCT GGC CTC CCC GAA GCG GCG GCA 40
CTT CAT GAC CCT CAG GTA CGT CTC CGT CTT ATG CAG GTC 79
CTT CCG GAA GCA GGA GAG CAG ACC GTA GTT CTT GAG CAG 118
CGC GTC GTC ACT GCG CAT GTT TGT GTC AAA TTT GTC ATA 157
GGT CTG CTT GAG GAT CTG CCC AGC CCG GGG GGT GCC ATC 196
TTC CAG CTC CCG CAT CAG GGC CAA GAT GCC TTC CTC CAG 235
GTC CTT CAG CTT CTC ATA GAC ACG GTC CGA GGT GCC AAA 274
CAC CAA GCT GTT GGT GAA GAC TCT GCT GAG AAA CTG CAG 313
GGG CCC AAG CCA CGA CTG GAT GAG GAG CAG TGA GAT GCG 352
AAG CAG CTC CAA GTC TGA TTT CTG CTG GGC CTC ATT CTT 391
GCC CGT GGG GGC CGG GAT GGT TTC GGA GAA GCA GAA GGC 430
AAC CTG GGT GTT CTG GAT GGA GTA TCT CTG TCC CTC CGG 469
GAT GTA GGT ACG CTC AAA CTC TTT GAA GGT GTC AGC AGC 508
CAG CTG GTG CAG GTG CTG AGC CCG GAG CAC AGC GTT GGC 547
AAA CAG GCC GGA CAA GGA CAT GGC TGG GAA CTT ATC ATC 586
ATC ATC CAA TGG GAA CA 603
```

## Claims

1. A process for conferring structural stability upon an expression vector which enables the production of a heterologous polypeptide from a host cell, said method comprising lowering the copy number of said expression vector within said host cell to a copy number at which the expression vector is structurally stable; wherein the gene encoding the heterologous polypeptide is expressed from a regulatable promoter.

2. A process according to Claim 1 wherein the copy number of the expression vector is lowered by insertion of a copy number control element into said expression vector.

3. A process according to Claim 2 wherein the copy number of the expression vector is lowered by insertion of the DNA encoding the rop gene into said vector, with the additional limitation that said vector contains a ColEI type origin of replication.

4. A process according to Claim 1 wherein the copy number of the expression vector is lowered by mutation of the host cell.

5. A process according to Claim 1 wherein the copy number of the expression vector is lowered by a mutation of said vector.

6. A process according to Claim 1 wherein the heterologous gene is selected from the group consisting of: human insulin A-chain; human insulin B-chain, human proinsulin; human insulin A-chain, B-chain and proinsulin analogs; human growth hormone; bovine growth hormone; EK-bovine growth hormone insulin-like growth factors; human transferrin; human interferons and human tissue plasminogen activators and derivatives thereof.

7. A process according to Claim 6 wherein the heterologous gene is selected from the group consisting of: human growth hormone; EK-bovine growth hormone and insulin-like growth factors.

8. A process according to Claim 2 wherein the heterologous gene is selected from the group consisting of: human insulin A-chain; human insulin B-chain; human proinsulin; human insulin A-chain, B-chain and proinsulin analogs; human growth hormone; bovine growth hormone; EK-bovine growth hormone; insulin-like grawth factors; human transferrin; human interferons and human tissue plasminogen activators and derivatives thereof.

9. A process according to Claim 8 wherein the heterologous gene is selected from the group consisting of: human growth hormone; EK-bovine growth hormone and insulin-like growth factors.

10. A process according to Claim 3 wherein he heterologous gene is selected from the group consisting of: human insulin A-chain; human insulin B-chain; human proinsulin; human insulin A-chain, B-chain and proinsulin analogs; human growth hormone; bovine growth hormone; EK-bovine growth hormone; insulin-like growth factors; human transferrin; human interferons and human tissue plasminogen activators and derivatives thereof.

11. A process according to Claim 10 wherein the heterologous gene is selected from the group consisting of: human growth hormone; EK-bovine growth hormone and insulin-like growth factors.

FIG. 1

**Restriction Site and Function Map of Plasmid pCZR125**

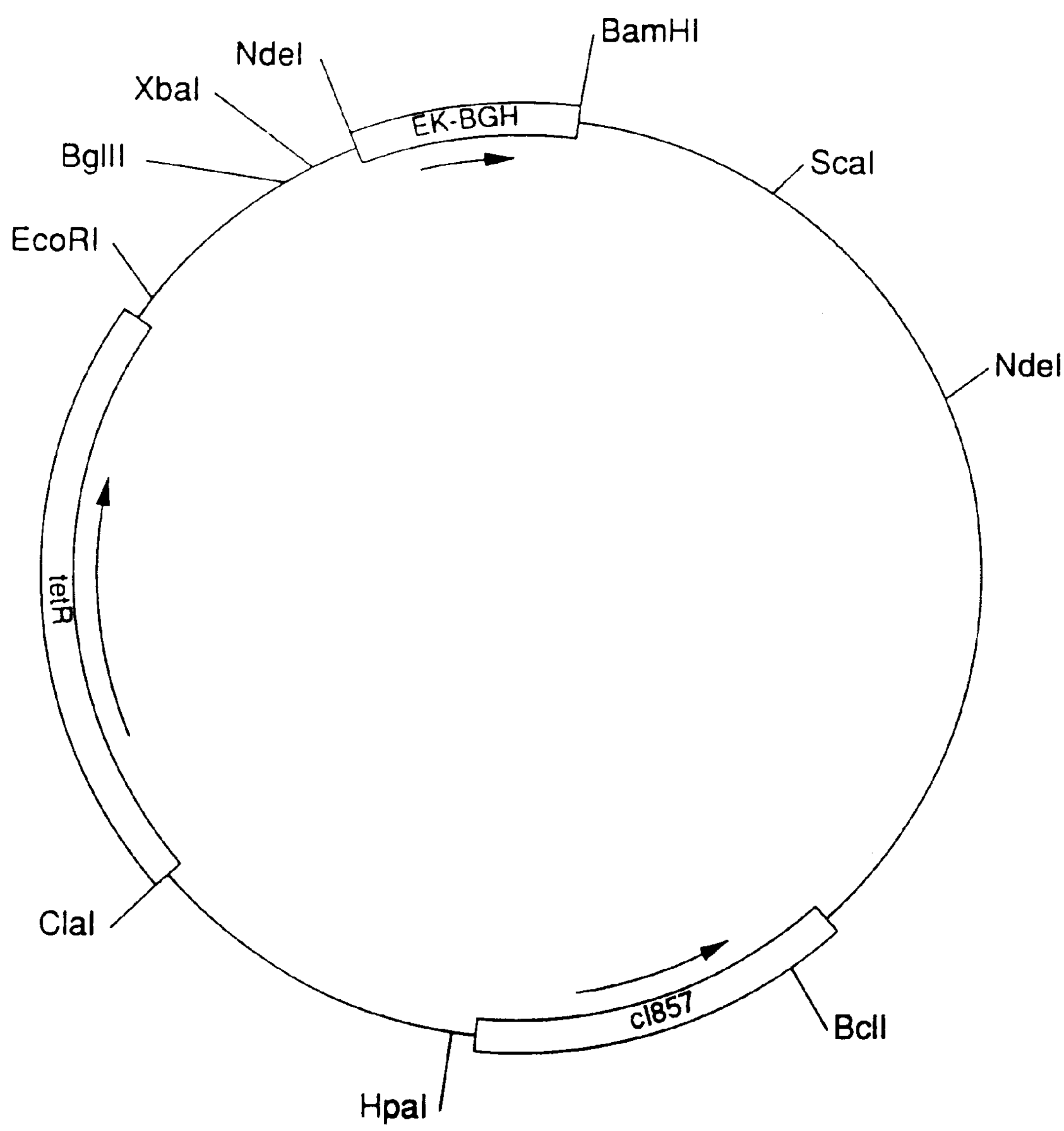

FIG. 2

**Restriction Site and Function Map of Plasmid pPR12**

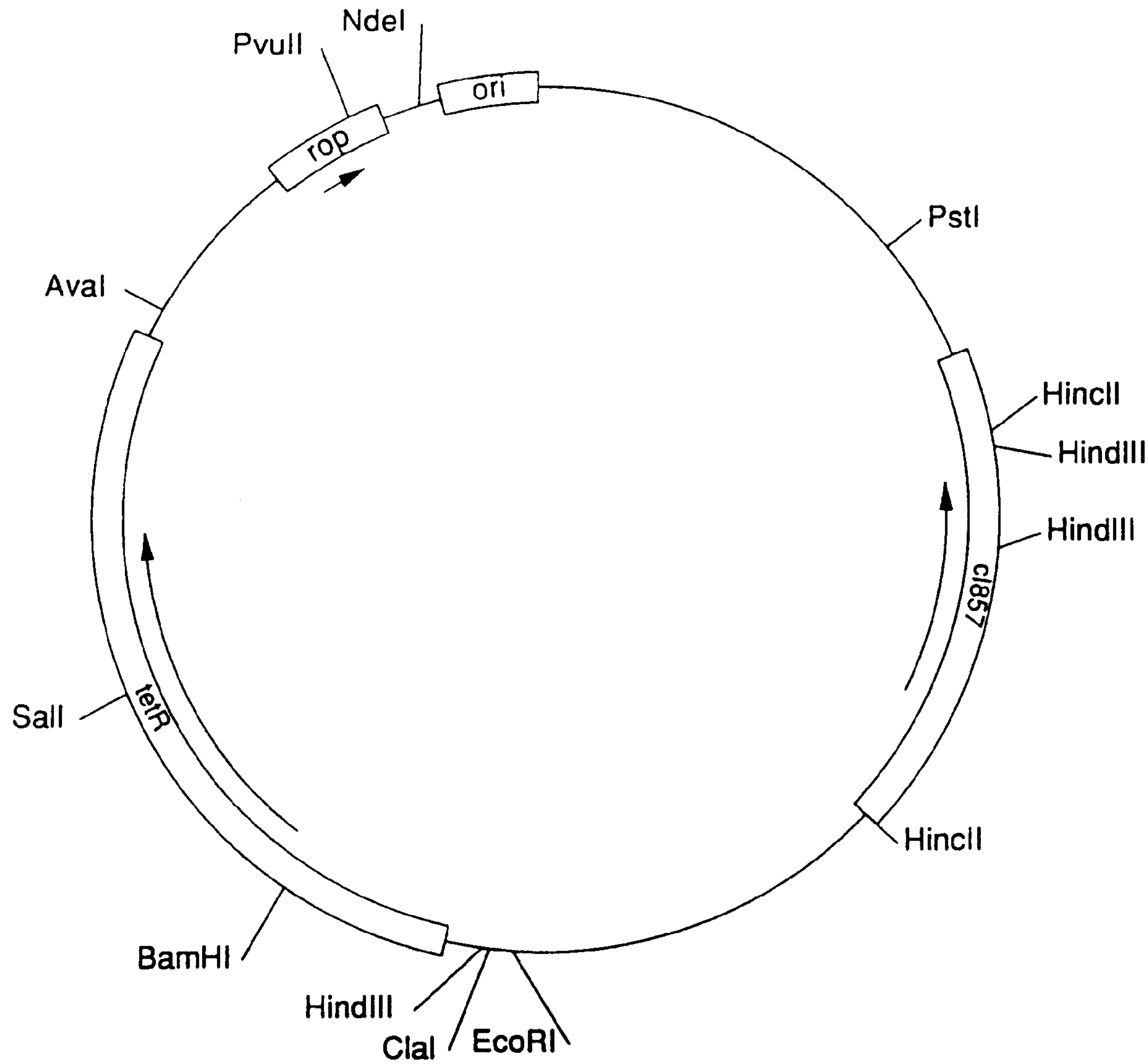

FIG. 3

**Restriction Site and Function Map of Plasmid pHPR91**

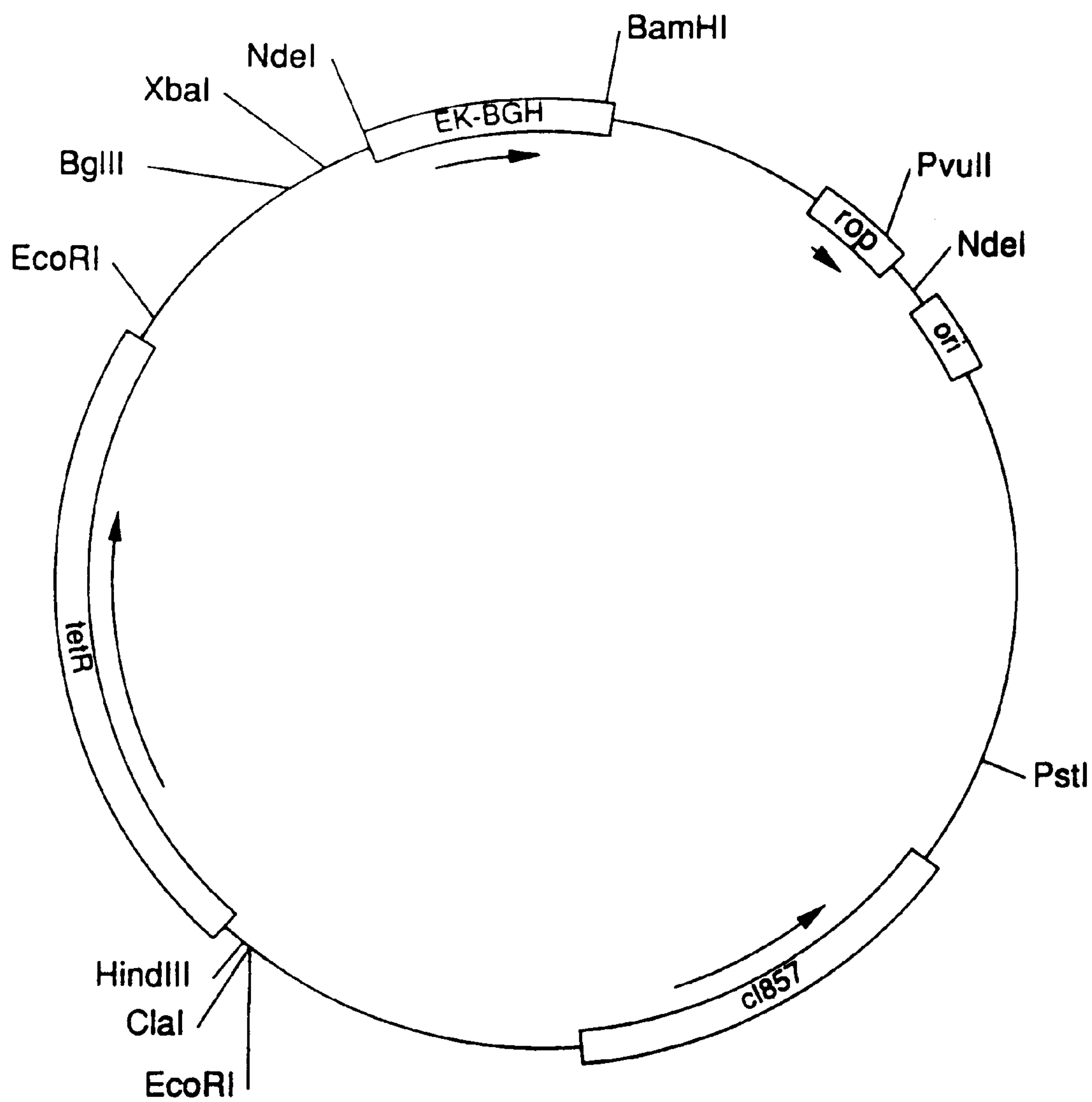

European Patent Office

# EUROPEAN SEARCH REPORT

Application Number

EP 91 31 1690
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GENE<br>vol. 94, no. 1, 28 September 1990, AMSTERDAM, NL<br>pages 121 - 124;<br>H. LEONHARDT: 'Identification of a low-copy-number mutation within the pUB110 replicon and its effect on plasmid stability in Bacillus subtilis'<br>* Whole article *<br>--- | 1,5 | C12N15/68<br>C12N15/70<br>C12N15/18 |
| X | BIOTECHNOLOGY AND BIOENGINEERING.<br>vol. 36, no. 9, November 1990, NEW YORK US<br>pages 865 - 878;<br>T.K. WOOD ET AL.: 'Depression of protein synthetic capacity due to cloned-gene expression in E. coli'<br>* Whole document, in particular page 875 column 1 *<br>--- | 1,5 | |
| X | JOURNAL OF BIOTECHNOLOGY<br>vol. 13, no. 1, January 1990, AMSTERDAM, NL<br>pages 47 - 60;<br>L. C. CHEW ET AL.: 'Simultaneous regulation of plasmid replication and heterologous gene expression in Escherichia coli'<br>* Whole article, in particular page 51 last paragraph - page 52 * | 1-3 | TECHNICAL FIELDS SEARCHED (Int. Cl.5 ) |
| Y | ---| 6-11 | C12N |
| D,Y | PROCEEDINGS OF THE NATIONAL ACADEMY OF SCIENCES OF USA.<br>vol. 83, no. 22, November 1986, WASHINGTON US<br>pages 8506 - 8510;<br>B.E. SCHONER ET AL.: 'Translation of a synthetic two-cistron mRNA in Escherichia coli'<br>* Whole article *<br>---<br>-/-- | 6-11 | |

The present search report has been drawn up for all claims

| Place of search | Date of completion of the search | Examiner |
|---|---|---|
| BERLIN | 21 APRIL 1992 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 91 31 1690
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | PROCEEDINGS OF THE NATIONAL ACADEMY OF SCIENCES OF USA. vol. 79, no. 20, October 1982, WASHINGTON US pages 6313 - 6317; G.CESARENI ET AL.: 'Control of ColE1 DNA replication: The rop gene product negatively affects transcription from the replication primer promoter' * Whole article * | 1-3 | |
| D,X | MGG (MOLECULAR & GENERAL GENETICS) vol. 205, no. 2, November 1986, BERLIN, G pages 285 - 290; J. LOPILATO ET AL.: 'Mutations in a new chromosomal gene of Escherichia coli K-12, pcnB, reduce plasmid copy number of pBR322 and its derivatives' * Whole article* | 1,4 | |
| X | EP-A-0 136 490 (AMGEN) * Page 8 lines 9 - 30, page 14 Table II and page 18 example 5 * | 1-2,6-9 | TECHNICAL FIELDS SEARCHED (Int. Cl.5) |
| X | EP-A-0 109 150 (A/S ALFRED BENZON) * Page 6 line 33 - page 8 line 27, page 12 line 22 - page 16 line 14 * | 1-2,6-9 | |
| D,A | DEVELOPMENTS IN INDUSTRIAL MICROBIOLOGY vol. 24, 1985, WASHINGTON DC, US pages 271 - 285; M.E. NUGENT ET AL.: 'The stability of recombinant DNA' * Whole article * | 1 | |

The present search report has been drawn up for all claims

| Place of search | Date of completion of the search | Examiner |
|---|---|---|
| BERLIN | 21 APRIL 1992 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)